Europäisches Patentamt

European Patent Office

Office européen des brevets

(19)

(11) Numéro de publication: **0 177 654**
**B1**

## (12) FASCICULE DE BREVET EUROPÉEN

(45) Date de publication du fascicule du brevet:
**31.05.89**

(21) Numéro de dépôt: **84402033.9**

(22) Date de dépôt: **10.10.84**

(51) Int. Cl.⁴: **A 61 K 31/565** //
(A61K31/565, 31:40)

(54) Association synergique contraceptive.

(43) Date de publication de la demande:
**16.04.86 Bulletin 86/16**

(45) Mention de la délivrance du brevet:
**31.05.89 Bulletin 89/22**

(84) Etats contractants désignés:
**AT BE CH DE FR GB IT LI LU NL SE**

(56) Documents cité:
**CHEMICAL ABSTRACTS, volume 84, no. 21, 24 mai 1976, page 87, abrégé 145226x, Columbus, Ohio, US; E. HERMAND et al.: "Hypothalamic mechanism of sulpiride antiovulatory action"**
**CHEMICAL ABSTRACTS, volume 91, no. 5, 30 juillet 1979, page 60, abrégé 33027w, Columbus, Ohio, US; J. BUVAT et al.: "The effect of sulpiride on LH and plasma progesterone in women of reproductive age. The role of hyperprolactinemia in causing ovulaton defects"**

(73) Titulaire: **SOCIETE D'ETUDES SCIENTIFIQUES ET INDUSTRIELLES DE L'ILE- DE- FRANCE, 46, Boulevard de Latour- Maubourg, F-75340 Paris Cédex 07 (FR)**

(72) Inventeur: **Howie, Peter William Pr., 8 Travebank Gardens, Monifieth Dundee DD5 4ET (GB)**
Inventeur: **McNeilly, Alan, 11 Craigend Road Stow, Galashiels Selkirkshire TDE 2RJ (GB)**

Il est rappelé que: Dans un délai de neuf mois à compter de la date de publication de la mention de la délivrance du brevet europeen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 177 654 B1

## Description

L'invention concerne une association synergique contraceptive, faite d'un benzamide hyperprolactinémiant, tel que le SULPIRIDE, ou N [(1-éthyl-2-pyrrolidinyl) méthyl] 2-méthoxy 5-sulfamoylbenzamide, et d'un progestatif de synthèse.

Le Sulpiride est essentiellement connu pour ses propriétés psychotropes. L'on à obsêrvé qu'à des doses usuelles en psychiatrie (100 à 1000 mg par jour), un effet contraceptif apparaissait. Toutefois, son usage à des fins de contrôle de la fertilité n'a pas été répandu en raison de la posologie relativement élevée nécessaire à une protection satisfaisante et des résultats d'essais cliniques concluant à une période de carence d'environ deux mois avant d'obtenir une contraception parfaite (D. BUVAT et al, Rev. Fr. Gynecol. Obstet. 71 (1) 53 - 61).

L'utilisation de psychotropes à des fins contraceptives n'a été à ce jour, préconisée que sous la forme d'application locales. Citons par exemple les demandes internationales de brevet de CORMIER (publications PCT 81/03 421 et 83/00 086) qui décrivent des préparations à usage intravaginal ou intra-uterin, comprenant un dérivé psychotrope, du type phénothiazine ou benzodiazépine par exemple, associé à une mousse, gelée ou autre véhicule pharmaceutique usuel à usage externe.

Le principe consiste à remplacer le composé spermicide usuel (ou à le compléter) par un psychotrope susceptible de pénétrer la membrane du spermatozoïde, et à empêcher ainsi son action de fécondation, en inhibant son activation par la calmoduline.

Il est connu d'utiliser des progestatifs de synthèse, à des fins contraceptives. On distingue trois types d'usage de ceux-ci:

1) utilisation quotidienne à très doses:

A faibles doses, ce procédé à pour effet de modifier la glaire cervicale et d'empêcher ainsi l'accessibilité des spermatozoïdes. A ces doses, il perturbe également la sécretion des gonadotrophines (FSH et LH) de façon variable suivant les sujets, entraînant les inconvénients suivants:

- efficacité non totale, surtout en début d'utilisation,
- saignements,
- suppression de l'hémorragie de privation (pseudo cycles menstruels normalement obtenus par un traitement intermittent), constituant un confort psychologique.

Citons le Norgestrel (à raison de 30 µg par jour) ou la Norethindrone (à raison de 300 µg par jour), comme exemples d'une telle utilisation.

2) utilisation pendant 21 jours par cycle, à des doses fortes:

L'effet contraceptif obtenu par l'administration de progestatifs selon ce procédé, relève du blocage de la secrétion des gonadotrophines, par exemple, par 2 mg (par jour) de diacétate d'éthynodiol pendant 21 jours. Il entraîne les inconvénients suivants:

- atrophie profonde et effets imprévisibles sur l'endomètre, conduisant à des métrorragies persistantes ou une absence de menstruations entre chaque cure,
- la tolérance clinique de la méthode est jugée très aléatoire et est mal acceptée par une proportion élevée de patientes.

3) utilisation sous forme "dépôt"

Ce procédé consiste en une injection mensuelle d'acétate de médroxyprogestérone (connu par exemple sous le nom de DEPO-PROVERA[R]), qui présente les inconvénients suivants, non réversibles tant que l'effet de l'injection se poursuit:

- disparition du cycle menstruel,
- saignements irréguliers ou prolongés,
- incertitude du moment de retour de la fertilité, après cessation du traitement.

Il est également bien connu, que nombre de préparations contraceptives associent un progestatif et un oestrogène (généralement l'éthinyloestradiol). Cependant, la partie oestrogénique de ces associations, est tenue pour responsable de certaines perturbations des métabolismes, et peut causer des accidents thrombo-emboliques. En conséquence on ne peut donc avoir recours dans tous les cas, à ce type de contraception, la fraction oestrogénique étant fortement contre-indiquée chez certaines patientes.

Il est donc proposé selon la présente invention une association synergique assurant dès le début une contraception efficace, à des doses faibles, ce qui permet de diminuer considérablemene les effets secondaires, comprenant:

- Un benzamide,

sélectionné en fonction de ses effets atténués sur le système nerveux central, et en fonction de puissants effets périphériques, notamment endocriniens; ces benzamides choisis parmi ceux qui ne sont pas cataleptigènes présentent la caractéristique d'être très actifs à faibles doses, sur le blocage de l'oestrus ce qui traduit un impact important sur l'axe hypothalamo-hypophysaire.

Citons par exemple le Sulpiride (1) qui à la dose de 1 mg/kg détermine un accroissement supérieur à 200 % du nombre de jours de dioestrus chez la ratte.

D'autres benzamides sont illustrés dans le tableau I:

(2) le N-(1-allyl 2-pyrrolidylméthyl) 2,3-diméthoxy 5-sulfamoyl benzamide

(3) le N-(1-éthyl 2-pyrrolidylméthyl-4-amino 5-éthylsulfonyl benzamide

(4) le N-(1-allyl 2-pyrrolidylméthyl) 2-méthoxy 4-amino 5-méthylsulfamoyl benzamide

(5) le N-(1-méthyl 2-pyrrolidylméthyl) 2-méthoxy 4-amino 5-éthylsulfonyl benzamide

- Un progestatif,

choisi de préférence parmi le Norgestrel, le diacétate d'Ethynodiol, la Médroxyprogestérone, la Norethindrone.

Chez les mères allaitantes, la lactation détermine une période d'infertilité d'abord très marquée, due à la complète inactivité ovarienne, dans la phase initiale du post-partum. Puis comme conséquence d'une diminution du nombre de tétées (et donc de stimulus du à la succion), une période d'infertilité relative, avec reprise d'une activité ovarienne, mais avec une phase lutéale anormale. Le mécanisme de cette infertilité fait encore l'objet d'investigations, mais on invoque l'élévation de la prolactine, consécutive à la tétée, comme cause possible.

La prolactine pourrait agir, sur l'hypothalamus en supprimant la rétroaction positive des oestrogènes responsables de l'ovulation, et en promouvant la rétroaction négative des stéroïdes gonadiques sur la sécrétion des gonadotrophines. D'autre part, la prolactine pourrait agir au niveau ovarien, en inhibant l'action des gonadotrophines sur la croissance folliculaire, les phases lutéales consécutives étant anormales du fait d'un mauvais développement folliculaire (Mc Neilly et al. J. Reprod. Fert. (1982) 65 - 559 - 569).

L'hypofertilité provoquée par certains benzamides, relève sans doute en partie de leur effet hyperprolactinémiant. En particulier, l'action antiovulatoire du sulpiride à été étudiée en fonction de l'hyperprolactinémie induite par administration de ce composé (J. Gynecol. Obstet. Biol. Reprod., 1978, 7 (1), 5 - 18). Cependant, il se peut que leur effet de blocage de l'oestrus ne dépende pas seulement du taux de prolactine dans le serum, mais aussi de l'inhibition de la sécrétion LH-RH. Un tel mécanisme est évoqué par Hermand et Coll. ("L'Encéphale" 1975 - I - 375 - 382).

Compte-tenu de ces observations et de l'effet inhibiteur connu des progestatifs sur la sécrétion des gonadotrophines, l'idée d'y associer un benzamide hyperprolactinémiant à faible dose est née. Les résultats cliniques ont montré qu'il se produisait alors un effet de synergie, permettant d'utiliser des doses moindres de chacun des composants (environ 5 à 25 mg de benzamide hyperprolactinémiant et 5 à 250 μg de progestatif).

La posologie quotidienne de 5 à 25 mg de Sulpiride (en moyenne 10 mg) associée à 5 à 250 μg de Noresthisterone prise comme exemple de progestatif, c'est-à-dire au maximum la moitié des doses usuelles, offre une contraception complète, avec une marge de sécurité accrue. L'intérêt d'une telle association est de proposer une "pilule starter", utilisable pendant un à trois mois en début de contraception, permettant de prévenir les inconvénients des contraceptifs précédemment décrits (en particulier assurant une immédiate sécurité et évitant les saignements fréquents), une contraception classique purement progestative prenant la suite, lorsque l'ovulation est bloquée de façon certaine.

Un autre intérêt présenté par l'invention réside dans la possibilité de l'utiliser parmi les populations allaitant pendant de longues durées, comme c'est le cas dans les pays en développement. En effet, la contraception oestroprogestative généralement prescrite aux mères allaitantes dans ces pays, tarit peu à peu la lactation. Cette inhibition de la lactation, combinée à une mauvaise utilisation fréquente de la contraception oestroprogestative peut causer une restauration prématurée de la fertilité et paradoxalement accroître le nombre des naissances.

Dans ce contexte, une association permettant à la fois une contraception sûre, et un maintien de la lactation, serait à considérer, d'autant que l'espacement des naissances et l'allaitement naturel sont des facteurs de lutte contre la mortalité infantile dans ces pays en développement.

Un troisième développement de l'invention présente un intérêt. En effet l'adjonction d'une très petite quantité d'oestrogène à l'association selon l'invention, permet d'obtenir une "pilule physiologique", c'est-à-dire d'établir un cycle mimant le cycle menstruel normal, avec quelques jours de saignement régulier lors de l'arrêt du traitement après trois semaines de prise. Outre une protection contraceptive complète, avec des doses très diminuées des substances en jeu (au maximum 15 μg d'éthinyl-oestradiol tandis que le contraceptif commercialisé le plus faiblement dosé en contient 30 μg), on obtient un confort psychologique du à des "menstruations" normales garantissant une prise régulière.

Alternativement, on peut utiliser un progestatif de synthèse tel que le Lynestrol ayant une action oestrogénique intrinsèque éliminant ainsi la nécessité d'un oestrogène.

Afin d'illustrer l'invention, de façon non limitative, les exemples suivants sont proposés:

**Exemple 1**: utilisation d'un progestatif

Dans une gélule, ou un comprimé inerte dont la fabrication est connue en soi, on introduit:

| | | |
|---|---|---|
| Norethisterone | 150 | μg |
| Sulpiride | 10 | mg |
| excipient | | qs 500 mg |

**Exemple 2**: utilisation d'un progestatif à activité oestrogénique

| | | |
|---|---|---|
| Lynestrol | 250 | μg |
| Sulpiride | 10 | mg |
| excipient | | qs 500 mg |

**Exemple 3**: utilisation d'un progestatif et d'un oestrogène

| | | |
|---|---|---|
| Norgestrel | 15 | µg |
| Sulpiride | 10 | mg |
| Ethinyloestradiol | 15 | µg |
| excipient | qs 500 mg | |

Toutes les formes galéniques usuelles, telles que par exemple comprimés, gélules, susceptibles de contenir les éléments de l'invention peuvent être utilisées pour son application.

La durée d'utilisation dépendra de l'appréciation du médecin, mais en général, un maximum de trois mois est recommandé, lorsqu'il s'agit d'utiliser l'invention comme précurseur de contraception progestative pure, classique, qu'elle soit sous forme d'absorption quotidienne (gélules, comprimés) ou sous forme d'administration "longue durée" (injection mensuelle par exemple).

**Tableau I**

| Composé à 1 mg/kg | augmentation du nombre de jours de dioestrus |
|---|---|
| 1 | + 231 % |
| 2 | + 168 % |
| 3 | + 251 % |
| 4 | + 164 % |
| 5 | + 157 % |

**Revendications**

1. Association contraceptive à effet synergique, comprenant 5 à 25 mg de benzamide hyperprolactinémiant et 5 à 250 µg de progestatif par unité galénique.

2. Selon la revendication 1, une association contraceptive dans laquelle le benzamide est le Sulpiride.

3. Selon la revendication 2, une association contraceptive comprenant 5 à 25 mg de sulpiride par unité galénique.

4. Selon les revendications 1 à 3, une association contraceptive dans laquelle le progestatif est la norethisterone.

5. Selon les revendications 1 à 4, une association contraceptive contenant un oestrogène dosé à moins de 30 µg par unité galénique.

6. Selon les revendications 1 à 5, une association contraceptive dans laquelle l'oestrogène est l'éthinyl-oestradiol.

7. Selon les revendications 1 à 3, une association contraceptive dans laquelle le progestatif est le Lynestrol.

8. Association contraceptive selon les revendications 1 à 7, utilisable comme précurseur temporaire de contraception progestative pure classique.

9. Association contraceptive selon les revendications 1 à 7, utilisable pendent la lactation sans nuire à son maintien.

**Patentansprüche**

1. Kontrazeptive Zusammensetzung mit synergistischer Wirkung, enthaltend 5 bis 25 mg eines hyperprolaktinämischen Benzamids und 5 bis 250 µg eines Gestagens pro galenischer Einheit.

2. Kontrazeptive Zusammensetzung nach Anspruch 1, in der das Benzamid Sulpirid ist.

3. Kontrazeptive Zusammensetzung nach Anspruch 2, enthaltend 5 bis 25 mg Sulpirid pro galenischer Einheit.

4. Kontrazeptive Zusammensetzung nach mindestens einem der Ansprüche 1 bis 3, in der das Gestagen Norethisteron ist.

5. Kontrazeptive Zusammensetzung nach mindestens einem der Ansprüche 1 bis 4, enthaltend ein Östrogen in einer Dosis von weniger als 30 µg pro galenischer Einheit.

6. Kontrazeptive Zusammensetzung nach mindestens einem der Ansprüche 1 bis 5, in der das Östrogen Ethinylöstradiol ist.

7. Kontrazeptive Zusammensetzung nach mindestens einem der Ansprüche 1 bis 3, in der das Gestagen Lynestrol ist.

8. Kontrazeptive Zusammensetzung nach mindestens einem der Ansprüche 1 bis 7 zur Verwendung als zeitlicher Vorläufer einer reinen, klassischen Gestagenkontrazeption.

9. Kontrazeptive Zusammensetzung nach mindestens einem der Ansprüche 1 bis 7 zur Verwendung während der Laktation ohne Beeinträchtigung des Verlaufs derselben.

**Claims**

1. Contraceptive combination with a synergic effect incorporating 5 to 25 mg of hyperprolactinemic benzamide and 5 to 250 µg of progestin per galenic unit.

2. A contraceptive combination according to claim 1, in which the benzamide is Sulpiride.

3. A contraceptive combination according to claim 2, incorporating 5 to 25 mg of Sulpiride per galenic unit.

4. Contraceptive combination according to claims 1 to 3, in which the progestin is Norethisterone.

5. Contraceptive combination according to claims 1 to 4, containing an estrogen in a quantity of less than 30 µg per galenic unit.

6. Contraceptive combination according to claims 1 to 5, in which the estrogen is ethinyl estradiol.

7. Contraceptive combination according to claims to 3, in which the progestin is Lynestrol.

8. Contraceptive combination according to claims 1 to 7, usable as a temporary precursor of conventional purely progestational contraception.

9. Contraceptive combination according to claims 1 to 7, usable during lactation without stopping the latter.